# EUROPÄISCHE PATENTSCHRIFT

(11) **EP 1 365 755 B1**
(45) Veröffentlichungstag und Bekanntmachung des Hinweises auf die Patenterteilung: **22.10.2008**
(21) Anmeldenummer: 02717980.3
(22) Anmeldetag: 27.02.2002
(51) Int. Cl.: A61K 31/7068, A61K 31/7072, A61P 31/12, A61P 31/18, A61K 31/70

(54) **DIE VERWENDUNG VON PYRIMIDINNUKLEOSIDEN UND/ODER PRODRUGS DAVON ZUR BEKÄMPFUNG VON NEBENWIRKUNGEN DER "HAART" (HIGHLY ACTIVE ANTI-RETROVIRAL THERAPY) UND ANDEREN ANTIVIRALEN THERAPIEN**
USE OF PYRIMIDINE NUCLEOSIDES AND/OR THE PRODRUGS THEREOF FOR COMBATING THE SIDE-EFFECTS OF HAART (HIGHLY ACTIVE ANTI-RETROVIRAL THERAPY) AND OTHER ANTI-VIRAL THERAPIES
UTILISATION DE NUCLEOSIDES PYRIMIDINIQUES ET/OU DE PRODROGUES DE CEUX-CI POUR LA LUTTE CONTRE DES EFFETS SECONDAIRES DE L'"HAART" (HIGHLY ACTIVE ANTI-RETROVIRAL THERAPY) ET D'AUTRES THERAPIES ANTIVIRALES

(30) Priorität: 03.03.2001 DE 10110355
(43) Veröffentlichungstag der Anmeldung: 03.12.2003
(73) Patentinhaber: Pharma Nord ApS, 6500 Vojens (DK)
(72) Erfinder: Walker, Ulrich, 79100 Freiburg (DE)
(74) Vertreter: Christensen, Bent
(86) Internationale Anmeldenummer: PCT/DE2002/000721
(87) Internationale Veröffentlichungsnummer: WO 2002/069943

(56) Entgegenhaltungen:
- WO-A-00/11952
- WO-A-00/50043
- WO-A-93/01202
- WO-A-93/12128
- DD-A- 114 411
- US-A- 5 968 914
- US-A1- 2001 005 719
- KAKUDA THOMAS N: "Pharmacology of nucleoside and nucleotide reverse transcriptase inhibitor-induced mitochondrial toxicity." CLINICAL THERAPEUTICS, Bd. 22, Nr. 6, Juni 2000 (2000-06), Seiten 685-708, XP002210767 ISSN: 0149-2918
- ASHOUR O. M., ET AL: "Modulation of 5-fluorouracil host toxicity by 5-(benzyloxybenzyl)barbituric acid acyclonucleoside, a uridine phosphorylase inhibitor, and 2',3',5'-tri-O-acetaluridine. a prodrug of uridine" BIOCHEMICAL PHARMACOLOGY, Bd. 60, Nr. 3, 2000, Seiten 427-431, XP001086586
- VENHOFF N. ET AL: 'Uridine pharmacokinetics of mitocnol, a sugar cane extract.' AIDS Bd. 19, 2005, Seiten 739 - 740
- WALKER U.A. ET AL: 'Beneficial effects of oral uridine in mitochondrial toxicity' AIDS Bd. 18, Nr. 7, 2004, Seiten 1085 - 1086
- WALKER U.A. ET AL: 'Uridine abrogates the adverse effects of antiretroviral pyrimidine analogues on adipose cell functions' ANTIVIRAL THERAPY Bd. 11, Nr. 1, 2006, Seiten 25 - 34
- SUTINEN JUSSI ET AL: 'Uridine supplementation for the treatment of antiviral therapy-associated lipoatrophy: a randomized, double-blind, placebo-controlled trial.' ANTIVIRAL THERAPY Bd. 12, Nr. 1, 2007, Seiten 97 - 105

## Beschreibung

Die Erfindung betrifft die Verwendung von Wirkstoffen, welche die Konzentration an Pyrimidinbasen-Bausteinen für die Nukleinsäurebiosynthese im Körper erhöhen, insbesondere von Pyrimidinnukleosiden und/oder Prodrugs davon, zur Verringerung von Nebenwirkungen von Hemmern der Biosynthese von Nukleinsäuren oder deren Vorstufen, insbesondere durch Aktivierung der Biosynthese mitochondrialer DNS (mtDNS), die Verwendung dieser Wirkstoffe, insbesondere von Pyrimidinnukleosiden und/oder Prodrugs, zur Herstellung von pharmazeutischen Präparaten zur Verringerung der genannten Nebenwirkungen, und die Verwendung der Kombinationen oder Produkte zur Verabreichung von derartigen Wirkstoffen, insbesondere von Pyrimidinnukleosiden und/oder Prodrugs davon, mit Hemmern der Biosynthese von Nukleinsäuren oder deren Vorstufen.

### Hintergrund der Erfindung

Unter der Bezeichnung "HAART" (Highly Active Anti-Retroviral Therapy) werden eine Reihe von Behandlungsmethoden zusammengefasst, bei denen antiretrovirale Chemotherapeutika, insbesondere Hemmstoffe der Reversen Transkriptase (RT), und Hemmstoffe der HIV-Protease, kombiniert zur Bekämpfung von AIDS verabreicht werden. Typische Behandlungsschemata verwenden zwei nukleosidanaloge RT-Hemmer (NRTIs) und einen HIV-Proteasehemmer oder einen nicht nukleosidanalogen RT-Hemmer. Nachdem zunächst vor allem Knochenmarkssuppression und damit einhergehend negative Auswirkungen einiger Hemmer der Reversen Transkriptase auf das Blutbild und die Immunabwehr als Nebenwirkungen festgestellt wurden, daneben auch zum Teil Neuropathien, Myopathien (auch am Herzmuskel), zeigten sich bei den neuen Therapieformen weitere Nebenwirkungen. Eine davon ist die sogenannte Lipodystrophie. Unter dieser Bezeichnung werden eine Reihe von Symptomen und Nebeneffekten zusammengefasst. Während mit Proteasehemmern oft hohe Cholesterin- und Triglyceridspiegel gefunden werden, werden mit den sogenannten nucleosidanalogen Reverse-Transkriptase-Hemmern (NRTIs) auch weitere Nebenwirkungen beobachtet. Augenfällig sind insbesondere die körperlichen Veränderungen, wie Schwund der Fettpolster im Gesicht, was zu eingefallenen Wangen und tiefliegenden Augen führen kann. Das Subkutanfettgewebe kann sich auch in anderen Körperregionen (z.B. an den Extremitäten) verringern. Zum Teil gleichzeitig hierzu, aber auch isoliert kann eine abnorme Fettgewebszunahme (z.B. an den Brüsten bei der Frau und beim Mann, intraabdominell und im Hals- und Nackenbereich auftreten. Insbesondere die letztgenannten Symptome sind eine Belastung für die Patienten, die darunter leiden, dass man ihnen an den Therapiefolgen "die Krankheit ansieht". Mögliche Folge der Fettveränderungen sind unter Umständen Stoffwechselveränderungen in anderen metabolischen Regulationskreisen wie z.B. der Glukosehomöostase durch Insulinresistenz und Veränderungen im Fettstoffwechsel und damit z.B. der Blutfettzusammensetzung. All diese Veränderungen werden unter dem Begriff Lipodystrophie zusammengefasst. Ein weiterer Effekt auch und besonders der NRTIs besteht in der Schädigung der Leber, insbesondere in Richtung auf eine Verfettung (mikro- oder makrovesikuläre Steatose, Steatohepatitis bis hin zu Leberversagen). Zudem zeichnen sich zahlreiche andere Syndrome ab, wie Veränderungen der Spermiogenese (verminderte Spermienanzahl, verminderte Spermienqualität, insbesondere verminderte Spermienmotilität), gehäufte Fälle von Pankreatitis und ein Syndrom der Osteopenie, welches mit der Laktaterhöhung assoziiert sein kann. Es werden ferner Funktionsstörungen der Nierentubuli beobachtet, wie z.B. eine verminderte Phosphatresorption. Letzlich weisen zahlreiche HIV-Patienten unter NRTI-haltiger antiretroviraler Langzeittherapie eine verminderte aerobe körperliche Leistungsfähigkeit aufgrund einer verminderten Sauerstoffaufnahme auf. Alle oben genannten Veränderungen scheinen eine Langzeit-Nebenwirkung der verschiedenen im Rahmen der HAART eingesetzten Wirkstoffe zu sein, wobei die meisten oben genannten Probleme solche der Langzeit-Therapie mit NRTIs zu sein scheinen.

Weil HAART jedoch sehr erfolgreiche Therapien zur Verfügung stellt, besteht grosser Bedarf an derartigen Behandlungsformen. Daher müssen dringend Möglichkeiten zur Verfügung gestellt werden, die neuen Nebenwirkungen, insbesondere Lipodystrophie, Störungen der Spermiogenese und Spermienfunktion, die Dysbalance des Mineralsalzgehaltes der Knochen (vor- und nachstehend Osteopenie), Leberschädigungen, das gehäufte Auftreten von Pankreatitis, die verminderte aerobe Leistungsfähigkeit und/oder die Nierenfunktionsstörungen, ferner Myopathien und/oder Schwächung von Zellen des Immunsystems, zu begrenzen, vermindern oder zu beseitigen. Bisher wurden hierfür, ohne nennenswerten Erfolg, vor allem Anpassungen in der Ernährungsweise zur Therapie und Prophylaxe gegen Nebenwirkungen eingesetzt, ferner Vitamin- und Mineralzufuhr und Zufuhr von ungesättigten Fettsäuren, Carnitin und dem Glutathionvorläufer N-Acetylcystein.

Eine weitere Nebenwirkung von NRTIs ist eine Hyperlaktatämie, welche bis zur Laktazidose, mit symptomatischen und zum Teil lebensbedrohlichen akuten Formen führen kann. Absetzen der NRTIs führt nur langsam zur Erholung, und die Verwendung von Kofaktoren oder Antioxidantien, wie Ubichinon, Antioxidantien, Carnitin, Riboflavin und Thiamin zeigt nur begrenzte Wirkung.

Die Behandlung aller genannten Nebenwirkungen ist insgesamt bisher eher unbefriedigend - das vorübergehende Absetzen von NRTIs und/ oder anderer Hemmer der Biosynthese von Nukleinsäuren oder deren Vorstufen ist wegen der Gefahr der Resistenzbildung und der Progression der HIV-assoziierten Immunschwäche meist nicht wünschenswert, wie oft auch ein Wechsel der Medikamente.

Patienten mit Infektionen durch andere Viren wie z.B. mit Infektionen durch das Hepatitis B-Virus, durch das Hepatitis C-Virus, durch das CMV-Virus, durch andere Herpesviren (wie z.B. Varizella-Zoster-Virus, und Herpes simplex Viren, Epstein-Barr Viren, HHV-Typ 6 und HHV-Typ 8) und durch das JC-Virus können ebenfalls mit Nukleosidanaloga (z.B. mit Acyclovir, Valacyclovir, Famaciclovir, Brivudin, Ribavirin, Ganciclovir, Tenofovir, Cidofovir und Adefovir) behandelt werden und erleiden potentiell die gleichen obigen Nebenwirkungen, bzw. durch die gleichzeitige Therapie mit gegen diese Viren gerichteten Nukleosidanaloga kann die Langzeitmedikation obiger HIV-Therapeutika verstärkt werden. Einzelne Nebenwirkungen können unter Verwendung dieser antiviralen Substanzen wesentlich ausgeprägter sein als unter NRTIs, z.B. Manifestierung der Nierenfunktionsstörung als Fanconi-Syndrom.

Daher besteht ein dringendes Bedürfnis nach neuen Wegen, die genannten unerwünschten Arzneimittelwirkungen, insbesondere Lipodystrophie, Spermienveränderung und/oder Osteopenie, ferner Pankreatitis, Nierenfunktionsstörung, verminderte aerobe Ausdauerleistung, Leberschädigung und/oder Laktazidose,wirksam zu bekämpfen, sowie ferner auch Myopathien zu bekämpfen und vorhandene Immunzellen zu reaktivieren.

In US 5,968,914 wird die Behandlung von durch AZT-induzierter Knochenmarkssuppression und damit einhergehender Anämie, sowie ddC-induzierter peripherer Neuropathie, Mundulzera, verminderter Thrombozytenzahl, und durch antivirale Therapie bedingter Nebenwirkungen in Muskel, peripherem Nervensystem, Immunsystemund Gastrointestinaltrakt mittels Gabe acyllerter nicht-methylierter Pyridinnucleoside beschrieben.
Die WO 00/11952 beschreibt unter anderem die Behandlung der Nebenwirkungen der Krebs-Chemotherapie, wie periphere Neuropathien, Nierenerkrankungen und Müdigkeit, durch Gabe von Pyrimidin-Nukleotid-Vorläufern.

WO-A-93/01202 beschreibt die Verwendung von Pyrimidinnukleosiden zur Behandlung von mitochondrialen Leiden, u.A. als Folge von Reverse-Transkriptase-Hemmern.

WO-A-00/50043 beschreibt die Verwendung von acylierten Pyrimidinnukleosiden zur Behandlung von Toxizität als Folge von Chemotherapie und antiviralen Mitteln. Kakadu, T.N. Clinical Therapeutics, Vol. 22(6), Seite 685-708, beschreibt mitochondrielle Toxizität induziert durch die Anwendung von NRTI.

WO-A-0011952 beschreibt die Verwendung von Pyrimidinnukleotiden in Zusammensetzungen und Methoden zur Behandlung von mitochondrialen Leiden.

US 2001/0005719 beschreibt die Verwendung von Pyrimidinnukleotid-Vorstufen in Zusammensetzungen und Methoden zur Behandlung von Mitochondrialen Leiden.

US 5,968,914 beschreibt die Verwendung von Pyrimidinnukleosiden in Zusammensetzungen und Methoden zur Behandlung und Prävention von Toxizität als Folge von Chemotherapie und antiviralen Mitteln.

### Allgemeine Beschreibung der Erfindung

Es wurde nun gefunden, dass eine große Anzahl von Nebenwirkungen von NRTIs und den oben aufgeführten anderen nukleosidanalogen antiviralen Medikamenten, insbesondere die Lipodystrophie, Spermienveränderung und/oder Osteopenle, ferner von Leberschädigung (insbesondere Steatohepatitis), Hyperlaktatämie/Laktazidose, Pankreatitis, Nierenfunktionsstörung und/oder verminderter aerober Ausdauerleistung und/oder ferner Schwächung vorhandener Zellen des Immunsystems wirksam mit Wirkstoffen, welche die Konzentration an Pyrimidinbasen-Bausteinen für die Nukleinsäurebiosynthese im Körper erhöhen, vorzugsweise Pyrimidinnukleosiden und/ oder Prodrugs davon, behandelt werden können.

Ohne durch diese Erklärung andere Wirkungsweisen und Mechanismen ausschließen zu wollen, lässt sich die Wirkung folgendermaßen plausibel erklären: NRTIs hemmen die für die mitochondriale DNS-Replikaton erforderliche γ-Polymerase und damit indirekt die Synthese mitochondrialer Atmungsketten-Untereinheiten. Diese Hemmung basiert einerseits auf der Kompetition der NRTIs mit den natürlichen mitochondrialen Nukleotiden, andererseits darauf, dass die NRTIs als Substrate für die Polymerase dienen und wegen ihrer fehlenden 3β-Hydroxygruppe einen Kettenabbruch bewirken. Die resultierende Verringerung der Menge mitochondrialer DNS (mtDNS) führt dann zu verminderter Synthese von durch diese mtDNS kodierten Untereinheiten der mitochondrialen Atmungskette. Dies führt nun dazu, dass auch die Aktivität der Dihydroorotat-dehydrogenase (DHODH), eines Enzyms, das an der de novo-Synthese von Pyrimidinnukleotden beteiligt ist, abnimmt. Der Grund ist, dass die Aktivität der DHODH an die Atmungskettenaktivität gekoppelt ist: Die Synthese von Orotat, einer Vorstufe für Pyrimidinnukleotide, ist nur möglich, wenn Elektronen von Dihydro-orotat auf Ubichinon (Coenzym Q) übertragen werden können. Dies geht kontinuierlich nur, wenn die Atmungskettenkomplexe III und IV funktionsfähig sind. Orotat wird dann normalerweise in Uridinmonophosphat umgewandelt, von dem aus alle anderen Pyrimidine biosynthetisiert werden. Daher ist anzunehmen, dass die Hemmung der Funktion der Atmungskette in Folge von der Gegenwart von NRTIs und/ oder anderer antiviraler nukleosidanaloger Substanzen, welche gegen andere Viren gerichtet sind, zu einer Verminderung der intramitochondrialen Pyrimidinnukleoside und - nukleotide führt. Als Folge davon nimmt die NRTI-Konzentration relativ zu der von Pyrimidinukleotiden zu, es kommt zu einer verstärkten Interaktion mit der γ-Polymerase. Ein Teufelskreis entsteht, der über weitere Verminderung der mtDNS zu noch weniger Pyrimidinnukleotidsynthese führt, usw. Die Wirkung der erfindungsgemäßen Zugabe von oben und unten beschriebenen Wirkstoffen, insbesondere Pyrimidinnukleosiden und/oder Prodrugs davon, durchbricht diesen Teufelskreis, indem eine exogene Quelle dieser Bausteine für mtDNS zur Verfügung gestellt wird. Damit gelingt es, die Nebenwirkungen der Nukleosidanaloga und anderer Hemmstoffe der Biosynthese von mitochondrialen Nukleinsäuren, insbesondere mtDNA, oder jeweils deren Vorstufen zu vermindern und insbesondere deren bereits beschriebene Langzeiteffekte zu bekämpfen.

Die Erfindung basiert daher auf der Verwendung von Wirkstoffen, welche die Konzentration an Pyrimidinbasen-Bausteinen für die Nukleinsäurebiosynthese im Körper erhöhen, insbesondere von Pyrimidinnukleosiden und/oder deren Derivaten, zur Beseitigung der unerwünschten Nebenwirkung Lipodystrophie von Hemmern der Biosynthese von Nukleinsäuren oder deren Vorstufen, insbesondere auf der Verwendung von Pyrimidinnukleosiden und/oder deren Derivaten zur (vollständigen oder teilweisen) Kompensation der durch diese Hemmer, insbesondere durch NRTIs und/oder andere antivirale Nukleosidanaloga, bedingten Verarmung an mtDNS und der damit einhergehenden Nebenwirkungen.

### Detaillierte Beschreibung der Erfindung

Die Erfindung betrifft (i) die Verwendung von Wirkstoffen, welche die Konzentration an Pyrimidinbasen-Bausteinen für die Nukleinsäurebiosynthese im Körper erhöhen insbesondere von Pyrimidinnukleosiden und/oder Prodrugs davon, zur Verringerung von der Nebenwirkung Lipodyshtrophie von Hemmern der Biosynthese von Nukleinsäuren oder deren Vorstufen, insbesondere von NRTIs und/oderanderen antiviralen Nukleosidanaloga, insbesondere die Verwendung von Pyrimidinnukleosiden und/oder deren Prodrugs zur Aktivierung der Biosynthese mitochondrialer DNS (mtDNS), d.h. zur (vollständigen oder teilweisen) Kompensation der durch diese Hemmer, insbesondere durch NRTIs und/oder andere antivirale Nukleosidanaloga, bedingten Verarmung (Depletion) von mtDNS (welche insbesondere auf Hemmung der mitochondrialen γ-Polymerase beruht) und der damit einhergehenden Nebenwirkungen, vor allem (ii) die Verwendung der Kombinationspräparate oder Produkte zur Verabreichung von derartigen Wirkstoffen, insbesondere von Pyrimidinnukleosiden und/oder Prodrugs davon, mit Hemmern der Biosynthese von Nukleinsäuren oder deren Vorstufen, zur Behandlung von der Nebenwirkung Lipodystrophie von Hemmern der Biosynthese von Nukleinsäuren oder deren Vorstufen, insbesondere durch Aktivierung der Biosynthese mitochondrialer DNS (mtDNS).

Die vor- und nachstehend verwendeten allgemeinen Begriffe und Symbole haben vorzugsweise die nachstehend genannten Bedeutungen, soweit nichts anderes angegeben ist. Soweit vor- und nachstehend allgemeine Begriffe und Symbole verwendet werden, können diese unabhängig voneinander durch die spezifischeren Definitionen ersetzt werden, was zu bevorzugten Ausführungsformen der Erfindung führt.

Die Verwendung kann prophylaktisch (d.h. zum vollständigen oder wenigstens teilweisen Vermeiden der genannten Nebenwirkungen vorher oder parallel zu einer Behandlung mit Hemmern der Biosynthese von Nukleinsäuren oder deren Vorstufen, beispielsweise bei HAART und/oder im Rahmen der Therapie anderer Virusinfektionen) oder, falls Nebenwirkungen bereits aufgetreten sind, therapeutisch erfolgen (beispielsweise parallel zu einer Therapie mit den genannten Hemmstoffen, oder in behandlungsfreien Zeitintervallen, z.B. (insbesondere im Falle der Behandlung akuter Laktazidose und/oder Hepatotoxizität) nach Absetzung der Hemmstoffe; dies alles ist von der vorliegenden Erfindung umfasst. In einer bevorzugten Ausführungsform erfolgt die erfindungsgemäße Verwendung von Wirkstoffen auch zur Behandlung, d.h. Vorbeugung und Therapie, der genannten Nebenwirkungen (insbesondere, soweit sie auf durch die antivirale Therapie bedingten Mitochondriopathien beruhen). beim Foetus und nach dessen Geburt, insbesondere im ersten Lebensjahr (z.B. beim Neugeborenen), im Rahmen der perinatalen Transmissionsprophylaxe mit Hemmern der Biosynthese von Nukleinsäuren oder deren Vorstufen.

Wirkstoffe, welche die Konzentration an Pyrimidinbasen-Bausteinen für die Nukleinsäurebiosynthese im Körper erhöhen, sind in erster Linie Verbindungen, die unter physiologischen Bedingungen in der Lage sind, eine Hemmung der Biosynthese mitochondrialer DNS (mtDNS) ganz oder wenigstens teilweise zu überwinden (Kompensation bzw. Aktivierung), wenn durch Behandlung mit Hemmern der Biosynthese von Nukleinsäuren oder von deren Vorstufen, insbesondere NRTIs und/oder anderen antiviralen Nukleosidanaloga, diese Synthese, insbesondere durch Hemmung der Dihydroorotat-dehydrogenase (DHODH), ganz oder teilweise unterbunden wird (d.h. insbesondere prophylaktisch vor, prophylaktisch oder therapeutisch während und/oder therapeutisch nach einer entsprechenden antiviralen Behandlung). Zu den Wirkstoffen gehören insbesondere solche Verbindungen, die in der Lage sind, die Konzentration an Orotsäure, Ribonucleosiden, Desoxyribonucleosiden, Ribonucleotiden und/oder Desoxyribonucleotiden, vor allem Uridin und ferner Cytidin, im Körper, insbesondere in Blutplasma, Zellen oder deren Organellen, wie Mitochondrien, zu erhöhen, was, wie oben beschrieben, eine (Re-)Aktivierung der Synthese von mtDNS ermöglicht. Hierzu gehören insbesondere Verbindungen wie Uridin-Phosphorylaseinhibitoren, Hemmstoffe der Uridinsekretion, Verbindungen, die mit Uridin bei renalen Transportmechanismen kompetitieren, oder Pyrimidinnukleoside und/ oder Prodrugs davon. Es ist auch möglich, zwei oder mehr der vor- und nachstehen genannten Wirkstoffe miteinander zu kombinieren, sei es in fester Kombination, sei es zeitlich gestaffelt.

Als Uridin-Phosphorylaseinhibitoren kommen insbesondere Verbindungen wie 5-Benzylbarbiturat-Derivate, wie 5-Benzylbarbiturat, 5-Benzyloxybenzylbarbiturat, 5-Benzyloxybenzyl- oder 5-Benzyloxybenzylacetyl-1-[(1-hydroxy-2-ethoxy)methyl]barbiturat, 5-Benzyloxybenzyl-1-[(1,3-dihydroxy-2-propoxy)methyl]barbiturat, 5-Benzyloxybenzyl-1-[(1-hydroxy-3-amino-2-propoxy)methyl]barbiturat, 5-Benzyloxybenzyl-1-[(2-(3-carboxypropionyloxy)ethoxy)methyl]-barbiturat, 5-Benzyl-1-[(1-hydroxy-2-ethoxy)methyl]barbiturat, 5-Methoxybenzylacetylbarbiturat, 5-Benzyl-1-[(1,3-dihydroxy-2-propoxy)methyl]barbiturat, 5-Benzyl-1-[(1-hydroxy-3-amino-2-propoxy)methyl]barbiturat, 5-Benzyl-1-[(2-(3-carboxypropionyloxy)ethoxy)methyl]barbiturat, 5-Methoxybenzylacetyl-acyclobarbiturat, 2,2'-Anhydro-5-ethyluridin, Acyclouridinverbindungen, wie 5-benzylsubstituierte Acyclouridinderivate, beispielsweise Benzylacyclouridin, Benzyloxy-benzyl-acyclo-uridin, Aminomethyl-benzyl-acyclouridin, Aminomethyl-benzyloxybenzyl-acyclouridin, Hydroxymethyl-benzylacyclouridin oder Hydroxymethyl-benzyloxy-benzyl-acyclouridin. Daneben sind weitere bekannt, z.B. in US 5,567,689 und den darin genannten Referenzen, insbesondere US 4,613,604, US 5,077,280 und/oder US 5,141,943.

Unter Hemmstoffen der Uridinsekretion werden solche Verbindungen verstanden, die den Transport von Uridin aus Zellen hemmen, insbesondere die renale Clearance von Uridin. Hierzu gehören insbesondere N,N'-bis[3-(3,4,5-trimethoxybenzoyloxy)propyl]homopiperazin (Dilazep) oder N,N'-Dimethyl)-N,N'-bis[3-(3',4',5'-trimethoxybenzoxy)propyl]ethylendiamin (Hexobendine), oder andere in US 5,567,689 genannte Verbindungen.

Verbindungen, die mit Uridin bei renalen Transportmechanismen kompetitieren, sind insbesondere L-Uridin, L-2',3'-Didesoxyuridin und D-2',3'-Didesoxyuridin.

Besonders bevorzugt sind (möglicherweise über auch weitere Mechanismen als die beschriebenen wirkende) Pyrimidinnukleoside und/oder Prodrugs davon

Pyrimidinnukleoside sind in erster Linie die Ribonucleoside oder die Desoxyribonukleoside von Uracil oder Cytosin, d.h. insbesondere Uridin und/oder Cytidin, ferner von Thymin, wie Thymidin.

Prodrugs sind in erster Linie metabolische Vorstufen der genannten Pyrimidinnukleoside, aus denen im Körper die Pyrimidinnukleoside hergestellt bzw. freigesetzt werden. Bevorzugt sind solche Verbindungen, bei denen eine oder mehrere der Hydroxygruppen im Riboserest mit jeweils einem Acylrest verestert sind und/oder, falls vorhanden, Aminogruppen (wie in Cytidin oder Desoxycytidin) mit Acylresten acyliert sind, oder Intermediate der Pyrimidinnucleotidbiosynthese, die in die Pyrimidin-Biosynthese distal der DHODH eintreten (wie Orotsäure oder deren Ester, beispielweise Alkylester)

Ferner können Prodrugs ausgewählt aus Triphenyluridin, Triphenylcytidin, Uridin- oder Cytidin-5'-monophosphat oder Prodrugs hiervon (z.B. Mono- oder Dialkylester, Acyloxyalkylester, Alkoxycarbonylmethylester, substituierte Ethyl- und Propylester, Amidomethylester, Benzylester, Phenylester, Phosphonoamidate, Cyclophosphatester, wie Cytidin-diphosphocholin oder Uridin-dipohosphoglukose); und Oligo- oder Polynucleotiden mit U und/oder C als Basenbausteinen, wie Homo- oder Heterodimere (z.B. U-P-U, U-P-C, C-P-U oder C-P-C, worin P für den verbindenden bivalenten Phosphorsäurerest steht), verwendet werden.

Wo von den Wirkstoffen, welche die Konzentration an Pyrimidinbasen-Bausteinen für die Nukleinsäurebiosynthese im Körper erhöhen, insbesondere Pyrimidinnukleosiden oder deren Derivaten, die Rede ist, beinhaltet dies, soweit die genannten Verbindungen salzbildende Gruppen tragen, diese Verbindungen in freier Form und/oder in Form von Salzen. Salze von Pyrimidinnukleosiden oder deren Prodrugs sind insbesondere die pharmazeutisch verwendbaren Salze. Liegen basische Gruppen vor, wie Amino oder Imino, können diese Säureadditionssalze bilden, beispielsweise mit anorganischen Säuren, wie Schwefelsäure oder Halogenwasserstoffen, oder mit organischen Säuren, beispielsweise Carbonsäuren, wie Essigsäure, oder Sulfonsäuren, wie Methansulfonsäure. Liegen saure Gruppen vor, wie Carboxy oder Sulfo, können diese Salze mit Kationen, beispielsweise von Metallen, wie Alkali- oder Erdalkalimetallen, z.B. Kalium oder Natrium, oder mit Amino- oder Ammoniumverbindungen, wie Ammoniumionen oder Niederalkylaminen, bilden. Auch innere Salze können gebildet werden, wenn sowohl saure als auch basische Gruppen vorliegen.

Halogen ist insbesondere Fluor, Chlor, Brom oder lod.

Das Präfix "Nieder" bedeutet, dass der betreffende Rest vorzugsweise bis zu 7, insbesondere bis zu 4 Kohlenstoffatome hat.

"Ferner" bedeutet "in einer breiteren, insbesondere weniger bevorzugten Ausführungsform der Erfindung".

"Die Konzentration m Körper erhöhen" bedeutet insbesondere eine Erhöhung der Konzentration in Blutplasma, Zellen oder deren Organellen, wie Mitochondrien, bei Warmblütern, vor allem Menschen.

Alkyl ist insbesondere C₁-C₂₅-Alkyl, vorzugsweise Niederalkyl. Die genannten Reste, wie auch Alkanoyl, Alkenoyl oder Alkinoyl, können linear oder ferner, falls es die Anzahl der C-Atome zulässt, in ein- oder mehrfach verzweigter Form vorliegen.

Acyl ist insbesondere die über Carbonyl an den bindenden Sauerstoff (oder, wie im Falle von Cytidin, ferner Stickstoff) gebundene Gruppe einer Carbonsäure (besonders bevorzugt) oder einer unsubstituierten oder substituierten Aminosäure (bevorzugt), ein über seine Carbonylgruppe an den bindenden Sauerstoff oder ferner Stickstoff geknüpfter Acylrest eines Halbesters der Kohlensäure (bevorzugt) oder ferner eine Aminocarbonylgruppe oder eine N-substituierte Aminocarbonylgruppe; Acyl hat vorzugsweise bis zu 25, in erster Linie bis zu 20 Kohlenstoffatome, wenn nicht anders angegeben.

Bevorzugtes Acyl einer Carbonsäure ist insbesondere unsubstituiertes oder substituiertes C₁-C₂₅-Alkanoyl, C₃-C₂₅-Alkenoyl oder C₃-C₂₅-Alkinoyl, insbesondere Niederalkanoyl, Octanoyl, Nonanoyl, Decanoyl, Undecanoyl, Dodecanoyl oder Palmitoyl, oder ferner substituiertes Niederalkanoyloxy, worin die Substituenten beispielsweise aus einem oder mehreren Resten, vorzugsweise ein bis drei Resten, insbesondere einem Rest, jeweils unabhängig voneinander ausgewählt aus Hydroxy, Niederalkoxy, Phenoxy, Naphthoxy, Niederalkanoyloxy, Phenylniederalkanoyloxy, wie Benzoyloxy oder Phenylacetyloxy, Halogen, wie Fluor, Chlor, Brom oder lod, insbesondere Fluor oder Chlor, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo, C₃-C₈-Cycloalkyl, wie Cyclopropyl, Cyclobutyl, Cyclopentyl oder Cyclohexyl, C₆-C₁₂-Bicycloalkyl, wie Decahydronaphth-2-yl, C₉-C₁₄-Tricycloalkyl,, wie 1- oder 2-Adamantyl, C₄-C₈-Cycloalkenyl, wie 1-Cyclohexenyl oder 1,4-Cyclohexadienyl, Heterocyclyl, welches vorzugsweise einen gesättigten, teilweise gesättigten oder ungesättigten einfachen Ring bedeutet, der 3 bis 7, vorzugsweuse 5 bis 7, Ringatome enthält, und bis zu vier Heteroatome ausgewählt aus Stickstoff, Schwefel und/oder Sauerstoff enthält, vorzugsweise 1 oder 2 der genannten Heteroatome; wobei der Ring entweder als solcher vorliegt oder bis zu zweifach, vorzugsweise einfach, benzanneliert, cyclopenta-, cyclohexa- oder cyclohepta-anneliert sein kann; und unsubstituiert oder, insbesondere durch Niederalkyl, Niederalkanoyl, Hydroxy, Niederalkoxy, Phenylniederalkoxy, wie Benzyloxy, Hydroxyniederalkyl, wie Hydroxymethyl, Halogen, Cyano und/oder Trifluormethyl, substituiert sein kann, z.B. Pyrrrolyl, 2,5-Dihydropyrrolyl, Furyl, Thienyl, Tetrahydrofuryl, Cyclohepta[blpyrrolyl, Pyrrolidinyl, Imidazolyl, Imidazolidinyl, Pyrazolinyl, Pyrazolidinyl, Triazolyl, wie 1,2,3-, 1,2,4-oder 1,3,4-Triazolyl, Tetrazolyl, wie 1- oder 2-Tetrazolyl, Tetrahydro-oxazolyl, Tetrahydroisoxazolyl, Tetrahydro-thiazolyl, Tetrahydro-Isothiazolyl, Indolyl, Isoindolyl, Chinolyl, Isochinolyl, Benzimidazolyl, Benzofuranyl, Pyridyl, Pyrimidinyl, Piperidinyl, Piperazin-1-yl, Morpholino, Thiomorpholino, S,S-Dioxothiomorpholino, 1,2-Dihydro- oder 1,2,3,4-Tetrahydrochinolyl, oder 1,2-Dihydro- oder 1,2,3,4Tetrahydroisochinolyl, wobei die genannten Reste unsubstituiert oder wie oben substituiert sind, insbesondere durch Niederalkyl, z. B. in 4-Niederalkyl-piperazin-1-yl, wie 4-Methyl- oder 4-Ethyl-piperazin-1-yl, durch Niederalkanoyl, z. B. in 4-Niederalkanoyl-piperazin-1-yl, wie 4-Acetyl-piperazin-1-yl, oder durch Hydroxyniederalkyl, z.B. in 5-Hydroxymethylfuran-2-ylcarbonyl, und Aryl, vorzugsweise C₆-C₁₄-Aryl, z.B. Phenyl, Naphthyl, wie 1- oder 2-Naphthyl, oder Fluorenyl, wie Fluoren-9-yl, wobei Aryl unsubstituiert oder beispielsweise durch Niederalkyl, z.B. Methyl, Halogenniederalkyl, wie Trifluormethyl oder Chlor oder Brommethyl, Halogen, z.B. Fluor oder Chlor, Hydroxy, Niederalkoxy, wie Methoxy, Niederalkanoyloxy, Carboxy, Niederalkyloxycarbonyl, Phenyiniederalkoxycarbonyl, Carbamoyl, Mono- oder Diniederalkylcarbamoyl, Mono- oder Dihydroxyniederalkylcarbamoyl, wie Heterocyclylniederalkyl, worin Heterocyclyl wie oben als Substituent von Niederalkanoyl definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazinl-ylmethyl, Morpholinomethyl oder-thiomorpholinomethyl, Cyano und/ oder Nitro ein oder mehrfach, vorzugsweise einfach, substituiert ist, insbesondere Phenyl, welches durch einen der genannten Reste in p-Stellung substituiert ist, ausgewählt sind, z.B. Niederalkanoyl, wie Formyl, Acetyl, Propionyl, Pivaloyl oder Heptanoyl, wie n-Heptanoyl, Hydroxyniederalkanoyl, z.B. β-Hydroxypropionyl, Niederalkoxyniederalkanoyl, z.B. Niederalkoxyacetyl oder Niederalkoxypropionyl, wie Methoxyacetyl oder β-Methoxypropionyl, Niederalkanoyloxyniederalkanoyl, z.B. Niederalkanoyloxyacetyl oder Niederalkanoyloxypropionyl, wie Acetoxyacetyl oder β-Acetoxypropionyl, Halogenniederalkanoyl, z.B. α-Halogenacetyl, wie α-Chlor-, α-Brom-, α-lod- α,α,α-Trifluar oder α,α,α-Trichloracetyl, oder Halogenpropionyl, wie β-Chlor- oder β-Brompropionyl, Carboxyniederalkanoyl, z.B. Carboxyacetyl oder 3-Carboxypropionyl, Niederalkoxycarbonylniederalkanoyl, z.B. Niederalkoxycarbonylacetyl oder Niederalkoxycarbonylpropionyl, wie Methoxycarbonylacetyl, β-Methoxycarbonylpropionyl, Ethoxycarbonylacetyl, β-Ethoxycarbonylpropionyl, tert-Butoxycarbonylacetyl oder β-tert-Butoxycarbonylpropionyl, Carbamoylniederalkanoyl, z.B. Carbamoylacetyl oder β-Carbamoylpropionyl, Niederalkylcarbamoylniederalkanoyl, Diniederalkylcarbamoylniederalkanoyl, Hydroxy-carboxy-niederalkanoyl, Hydroxy-niederalkoxycarbonyl-niederalkanoyl, Dihydroxycarboxynieder alkanoyl, Dihydroxy-niederalkoxycarbonyl-niederalkenoyl, Heterocyclylniederalkanoyl, beispielsweise Pyrrolylcarbonyl, wie 2- oder 3-Pyrrolylcarbonyloxy, Furylcarbonyl, z.B. 2-Furylcarbonyl, 5Hydroxymethyl-furan-2-ylcarbonyl, Thienylcarbonyl, z.B. 2-Thienylcarbonyl, Imidazolocarbonyl, wie 4-Imidazolylcarbonyl, Imidazolylacetyl, wie 4-Imidazolylacetyl, Imidazolylpropionyl, wie 3-(4-Imidazolylpropionyl, Pyridylcarbonyl, z.B. 2-, 3- oder 4-Pyridylcarbonyl, Indolylcarbonyl, z.B. 2-, 3- oder 5-Indolylcarbonyl, 1-Methyl-, 5-Methyl-, 5-Methoxy-, 5-Benzyloxy-, 5-Chlor- oder 4,5-Dimethylindolyl-2-carbonyl, Chinolylcarbonyl, wie Chinolin-2-ylcarbonyl, Pyrrolidinylcarbonyl, wie Pyrrolidinyl-3-carbonyl, Piperidinylcarbonyl, z.B. 2-, 3- oder 4-Piperidinylcarbonyl, Morpholino-carbonyl, Thiomorpholinocarbonyl, Morpholinoacetyl, Thiomorpholinoacetyl, oder 4-Niederalkyl-piperazinoacetyl, wie 4-Methyl-piperazinoacetyl, Niederalkenoyl, z.B. Acryloyl, Vinylacetyl, Crotonoyl oder 3- oder 4-Pentenoyl, Niederalkinoyl, z.B. Propinoyl oder 2- oder 3-Butinoyl, C₃-C₈-Cycloalkylcarbonyl oder C₃-C₈-Cycloalkylacetyl, z.B. Cyclopropyl-, Cyclobutyl-, Cyclopentyl- oder Cyclohexylcarbonyl, Cyclopropylacetyl, Cyclopentylacetyl oder Cyclohexylacetyl, Phenylniederalkanoyl, z.B. Benzoyl, Phenylacetyl oder 3-Phenylpropionyl, worin Phenyl unsubstituiert, mono- oder mehrfach substituiert durch Niederalkyl, z.B. Methyl, Haloniederalkyl, wie Chlor- oder Brommethyl, Halogen, z. B. Fluor oder Chlor, Hydroxy, Niederalkoxy, z.B. Methoxy, Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinoniederalkyl, wie Morpholinomethyl, Thiomorpholinoniederalkyl, wie -methyl, Cyano und/ oder Nitro, z.B. 4-Chlormethyl-, 4-Brom-methyl-, 4-Fluor-, 4-Chlor-, 4-Methoxy-, 4-Morpholinomethyl-, 4-Thiomorpholinomethyl-, 4-Cyano- oder 4-Nitrobenzoyl, oder Niederalkylphenylacetyl, wie 4-Methylphenylacetyl. Besonders bevorzugt sind die Acylreste unsubstituierter Carbonsäuren, wie oben definiert, oder die Acylreste physiologisch vorliegender Carbonsäuren, wie Glykoloyl, Lactoyl, Enolpyruvoyl, Liponoyl, Pantothenoyl, Acetoacetoyl, p-Aminobenzoyl, β-Hydroxybutyroyl, Creatinoyl oder Orotoyl.

Bevorzugtes Acyl eines über seine Carbonylgruppe an den bindenden Sauerstoff geknüpften Acylrestes eines Halbesters der Kohlensäure ist z.B. unsubstituiertes oder substituiertes Hydrocarbyloxycarbonyl, vorzugsweise mit 2 bis 20 C-Atomen, insbesondere unsubstituiertes oder ferner substituiertes Niederalkoxycarbonyl, z. B. Methoxy-, Ethoxy- oder tert-Niederalkoxycarbonyl, wie tert-Butoxycarbonyl, 2-Halogenniederalkoxycarbonyl, z.B. 2-Chlor-, 2-Brom-, 2-lod- oder 2,2,2-Trichlorethoxycarbonyl; Arylniederalkoxycarbonyl, z.B. Arylmethoxycarbonyl, worin Aryl vorzugsweise 6 bis 14 Kohlenstoffatome hat, unsubstituiert oder beispielsweise durch Niederalkyl, z.B. Methyl, Halogenniederalkyl, wie Trifluormethyl oder Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Heterocyclylniederalkyl, worin Heterocyclyl wie oben als Substituent für Niederalkanoyl definiert ist, insbesondere Heterocyclylmethyl, worin Heterocyclyl über ein Ringstickstoffatom gebunden ist, Piperidinomethyl, Piperazin-1 - ylmethyl, 4-Niederalkyl-piperazin-1-ylmethyl, wie 4-Methyl- oder 4-Ethylpiperazin-1-ylmethyl, 4-Niederalkanoyl-piperazin-1-ylmethyl, wie 4-Acetyl-piperazin-1-ylmethyl, Morpholinomethyl oder Thiomorpholinomethyl, Cyano und/oder Nitro ein- oder mehrfach, vorzugsweise einfach, substituiert ist, und insbesondere Phenyl, 1- oder 2-Naphthyl, Fluorenyl oder durch Niederalkyl, z.B. Methyl oder tert-Butyl, Niederalkoxy, z.B. Methoxy, Ethoxy oder tert-Butoxy, Hydroxy, Halogen, z.B. Fluor, Chlor oder.Brom, und/oder Nitro mono- oder mehrfach substituiertes Phenyl ist, z.B. Phenylniederalkoxycarbonyl, wie Benzyloxycarbonyl, 4-Methoxybenzyloxycarbonyl, 4-Nitrobenzyloxycarbonyl, Diphenylniederalkoxycarbonyl, wie Diphenylmethoxycarbonyl, Di-(4-methoxyphenyl)-methoxycarbonyl, Trityloxycarbonyl oder Fluorenylniederalkoxycarbonyl, wie 9-Fluorenylmethoxycarbonyl; oder ferner Heterocyclylniederalkoxycarbonyl, worin Heterocyclyl wie oben als Substituent von Niederalkanoyl definiert ist, z.B. Furan-2-ylmethoxycarbonyl oder Pyridin-2-, -3- oder -4-ylmethoxycarbonyl.

Eine bevorzugte N-substituierte Aminocarbonylgruppe als Acyl trägt am Stickstoff 1 bis 2 Substituenten, die unabhängig voneinander aus unsubstituiertem oder substituiertem Niederalkyl, wobei die Substituenten aus den oben für substituiertes Niederalkanoyl genannten ausgewählt sind und in der dort definierten Anzahl vorhanden sein können, vorzugsweise Substituenten ausgewählt aus Hydroxy, Niederalkoxy, Niederalkanoyloxy, Phenylniederalkenoyloxy, wie Benzoyloxy oder Phenylacetyloxy, Halogen, wie Fluor, Chlor, Brom oder lod, insbesondere Fluor oder Chlor, Carboxy, Niederalkoxycarbonyl, Phenylniederalkoxycarbonyl, wie Benzyloxycarbonyl, Cyano, Oxo und Phenyl oder Naphthyl, welche unsubstituiert oder beispielsweise durch Niederalkyl, z.B. Methyl, Halogenniederalkyl, wie Trifluormethyl oder Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Cyano und/oder Nitro ein oder mehrfach, vorzugsweise einfach, substituiert sind, insbesondere Phenyl, welches durch einen der genannten Reste in p-Steilung substituiert ist; insbesondere aus unsubstituiertem Niederalkyl, wie Methyl oder Ethyl; und Aryl, welches vorzugsweise 6 bis 14 Kohlenstoffatome hat und unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Haloniederalkyl, wie Trifluormethyl, Cyano und/oder Nitro ein oder mehrfach, vorzugsweise einfach, substituiert ist, ausgewählt sind, wobei der Stickstoff der Carbamoylgruppe nicht mehr als einen Arylrest trägt. Die jeweils unter die Definition von Acylgruppen einer N-substltuierten Carbaminsäure fallenden Definitionen und der Rest Aminocarbonyloxy können vorzugsweise weggelassen werden.

Eine über ihr Carbonyl an den bindenden Sauerstoff gebundene unsubstituierte oder substituierte Aminosäure in Acyl wird vorzugsweise gebildet durch die über das Carbonyl ihrer Carboxygruppe und ein Sauerstoffatom gebundenen Aminosäurereste (Aminoacyl) einer α-, β-, γ- oder δ-Aminosäure, insbesondere einer natürlichen α-Aminosäure mit der L-Konfiguration, wie sie normalerweise in Proteinen vorkommen, oder eines Epimeren einer solchen Aminosäure, d. h. mit der unnatürlichen D-Konfiguration, oder deren D,L-Isomerengemisch, eines Homologen einer solchen Aminosäure, z. B. worin die Aminosäurenseitenkette um eine oder zwei Methylengruppen verlängert oder verkürzt ist, worin die Aminogruppe in β-, γ- oder δ-Stellung vorliegt und/oder worin eine Methylgruppe durch Wasserstoff ersetzt ist, einer substituierten aromatischen Aminosäure, worin der aromatische Rest 6 - 14 Kohlenstoffatome hat, z. B. eines substituierten Phenylalanins oder Phenylglycins, worin der Phenyl-Substituent Niederalkyl, z. B. Methyl, Hydroxy, Niederalkoxy, z.B. Methoxy, Niederalkanoyloxy, z. B. Acetoxy, Amino, Niederalkylamino, z. B. Methylamino, Diniederalkylamino, z. B. Dimethylamino, Niederalkanoylamino, z. B. Acetylamino oder Pivaloylamino, Niederalkoxycarbonylamino, z. B. Kohlenstoffatome hat, z. B. Benzyloxycarbonylamino oder 9-Fluorenylmethoxycarbonylamino, Halogen, z. B. Fluor, Chlor, Brom oder lod, Carboxy und/oder Nitro sein kann und ein- oder mehrfach vorkommt, eines benzannellierten Phenylalanins oder Phenylglycins, wie α-Naphthylalanins, oder eines hydrierten Phenylalanins oder Phenylglycins, wie Cyclohexylalanins oder Cyclohexylglycins.

Diese Aminosäurereste können an freien Amino- oder Hydroxyfunktionen substituiert sein, wie oben für Aminosäurereste R, oder Rg beschrieben. Besonders bevorzugt ist das über das Carbonyl seiner Carboxygruppe und ein Sauerstoffatom gebundene Radikal einer Aminosäure ausgewählt aus Glycin, Alanin, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure oder 5-Aminohexansäure, Valin, Norvalin, Leucin, , Isoleucin, Norleucin (α-Aminohexansäure), Serin, Homoserin (α-Amino-γhydroxybuttersäure), Threonin, Methionin, Cystein, Prolin, trans-3- und trans-4-Hydroxyprolin, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Indolin-2-carbonsäure, 1,2,3,4-Tetrahydroisochinolin-3-carbonsäure, Asparaginsäure, Asparagin, Aminomalonsäure, Aminomalonsäure-monoamid, Glutaminsäure, Glutamin, Histidin, Arginin, Lysin, 8-Hydroxylysin, Ornithin, 3-Aminopropansäure, α,γ-Diaminobuttersäure und α,β-Diaminopropionsäure, besonders bevorzugt der Rest einer aliphatischen Aminosäure ausgewählt aus Alanin, Valin, Norvalin, Leucin, 3-Aminopropionsäure, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure oder 5-Aminohexansäure und Isoleucin oder einer Aminosäure ausgewählt aus Glycin, Asparagin, Glutamin, Methionin, Lysin, Histidin, Prolin, Carnitin und Phenylalanin, wobei (ausser in Fällen, wo kein asymmetrisches Kohlenstoffatom vorliegt, z.B. bei Glycin) jede der genannten Aminosäuren in der D-, L- oder (D,L)-, vorzugsweise in der L-Form vorliegen kann, und eine Aminogruppe unsubstituiert oder ein- oder zweifach, oder dreifach, N-alkyliert, z. B. durch Niederalkyl, wie Methyl, n-Propyl oder n-Butyl, durch Pyridylniederalkyl, wie 2-,3- oder 4-Pyridylmethyl, und/oder durch Phenylniederalkyl, wie Benzyl, wobei der Carnitinrest bevorzugt ist, und/oder N-acyliert, z.B. durch unsubstituiertes oder substituiertes Niederalkanoyl, wie oben für Niederalkanoyl, definiert, vor allem durch Acetyl, Propionyl oder Pivaloyl, Arylniederalkanoyl, z.B. Phenylniederalkanoyl, wie Benzoyl oder Phenylacetyl, durch Niederalkoxycarbonyl, wie tert-Butoxycarbonyl, oder durch Arylniederalkoxycarbonyl, z.B. Phenylniederalkoxycarbonyl, wie Benzyloxycarbonyl; vorliegt. Von den zuletzt genannten Resten sind Acylgruppen einer unsubstituierten oder substituierten Aminosäure ausgewählt aus Aminoacetyl (Glycyl), N-Niederalkylaminoacetyl, N,N-Diniederalkylaminoacetyl, N-Niederalkyl-N-phenylniederalkylaminoacetyl, N-Niederalkyl-N-niederalkoxycarbonylaminoacetyl und N-Phenylniederalkoxycarbonyl-N-niederalkylaminoacetyl, z.B. N-Methylaminoacetyl, N,N-Dimethylaminoacetyl, N-Methyl-N-(n-butyl)aminoacetyl, N-Methyl-N-benzylaminoacetyl, N-Methyl-N-[(2-, 3- oder 4-)pyridylmethyl-aminoacetyl, wie N-Methyl-N-3-pyridylmethylaminoacetyl, N-Methyl-N-tert-butoxycarbonylaminoacetyl, N-Benzyloxycarbonyl-N-niederalkylaminoacetyl, Prolyl, Histidyl, Glutamyl, Asparagyl und Carnitinoyl bevorzugt, wobei die Aminosäurereste (ausser in Fällen, wo kein asymmetrisches Kohlenstoffatom vorliegt, z.B. bei Gly) vorzugsweise in der (L)-, ferner der (D)- oder (D,L)-Form vorliegen.

Die erfindungsgemäß verwendbaren Prodrugs oder Pyrimidinnukleoside sind insbesondere solche der Formeln I (Uridin oder Prodrugs davon) oder II (Cytidin oder Prodrugs davon), 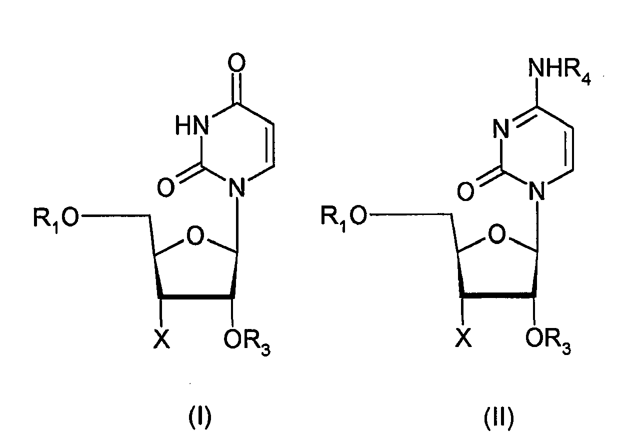 worin X für Wasserstoff oder -OR₂ steht und R₁, R₂, R₃ und, falls vorhanden, R₄ unabhängig voneinander aus Wasserstoff und Acyl (vorzugsweise den als bevorzugt beschriebenen Acylresten) ausgewählt sind.

Die erfindungsgemäß verwendbaren Pyrimidinnukleoside oder Prodrugs sind bekannt, kommerziell erhältlich oder nach an sich bekannten Verfahren herstellbar, z.B. nach oder analog den in US 5,968,914, US 5,583,117, US 5,567,689, WO 00/11952 oder EP 0 604 368 gegebenen Vorschriften.

Unter Hemmern der Biosynthese von Nukleinsäuren (wie DNS oder RNS) oder deren Vorstufen (beispielsweise Nukleotide, Desoxynukleotide oder jeweils die Mono-, Di- oder Triphosphate davon) sind insbesondere
- Hemmstoffe der Ribonucleosiddiphosphatreduktase, vor allem Hydroxyharnstoff (Hydroxycarbamid; z.B. Litalir^{®}, Bristol-Myers Squibb), Didox oder Trimidox
- Hemmstoffe der Purinbiosynthese, wie 2-Morpholinoethyl-(E)-6-(1,3-dihydro-4-hydroxy-6-methoxy-7-methyl-3-oxoisobenzofuran-5-yl)-4-methyl-4-hexenoat (Mycophenolat.Mofetil; z.B. CellCept^{®}, Hoffmann-LaRoche);
- Hemmer der Dihydrofolat-Reduktase, z.B. Methotrexat, Pyrimethamin, Proguanil, Cycloguanil oder Trimethoprim; oder
- vorzugsweise Hemmstoffe der mitochondrialen γ-Polymerase, insbesondere Hemmstoffe der reversen Transkriptase (RT), beispielsweise von HIV, insbesondere nukleosidanaloge-RT-Hemmer (NRTIs), in erster Linie AZT* (Zidovudin = 3'-Azido-3'-desoxythymidin; z.B. Retrovir^{®}; Glaxo-SmithKline), Didanosin* (ddl, 2',3'-Didesoxyinosin; z.B. Videx^{®}; BMS), Zalzitabin* (ddC, Didesoxycytidin; z.B. HIVID Roche^{®}), Lamivudin* (2'-Desoxy-3'-thiacytidin, 3TC; z.B. Epivir^{®}; Glaxo-SmithKline), Stavudin* (d4T = 2',3'-Didehydro-2',3'-didesoxythymidin,z.B. Zerit^{®}; BMS), Abacavir (z.B. Ziagen^{®}; Glaxo-SmithKline); oder d4C (2',3'-Didehydro-2'.3'-didesoxycytidin),; oder ferner FTC (Emtricitabin, Covirazil = fluoridiertes 3TC; Triangle/ Abbott); DAPD (Guanosinanaloges; Triangle/Abbott) sowie sein deaminierter Metabolit DXG; Fozivudin tidoxil (Thioether-Lipid-Zidovudin-Konjugat); ACH-126,443 (b-L-Fd4C, Achillion); Fosphazid (Phosphonavir, PZT); Tenofovir* (PMPA), Lodenosin (FddA, ddA), BCH-10618 (BioChemPharm), BCH-20652/dOTC (3TC-Verwandter, BioChemPharm), BCH-13520, wobei die mit Sternchen ("*") markierten Verbindungen jeweils bevorzugt sind;
- oder ferner andere antivirale Nukleosidanaloga (Hemmstoffe weiterer Viren), z. B. nukleosidanaloge Hemmstoffe des Hepatitis B-Virus, des Hepatitis C-Virus, des CMV-Virus, anderer Herpesviren, wie z.B. Varizella-Zoster-Virus, der Herpes simplex Viren, des Epstein-Barr Virus, des HHV-Typ 6 und des HHV-Typ 8) sowie des JC-Virus (z.B. Acyclovir, Penciclovir, Sorivudin, Valacyclovir, Famciclovir (z.B. Famvir; Glaxo-SmithKline), Brivudin (z.B. Helpin^{®}, Berlin-Chemie), Entecavir (BMS-200475), Trifluridin (TFT; z.B. Thilo^{®}), Idoxuridin (IDU; z.B. Synmiol^{®}),in erster Linie aber Fialuridin, Ribavirin (z.B. Rebetol^{®}; Essex), Ganciclovir (z.B. Cymeven^{®}), Cidofovir (z.B. Vistide^{®}) und Adefovir-Dipivoxil (z.B. Adefovir^{®}; Gilead).
- oder Kombinationen von 2 oder mehr dieser Hemmstoffe zu verstehen, und/oder
- ferner Kombinationen eines der vorgenannten Hemmstoffe mit Inhibitoren der Dihydroorotsäure-Dehydrogenase, z.B. mit Leflunomid (z.B. Arava^{®}; Aventis; hier ist es aufgrund der langen Halbwertszeit insbesondere möglich, auch Zellen des Immunsystems einige Tage nach Verabreichung von Leflunomid durch erfindungsgemäße Wirkstoffverabreichung zu reaktivieren) oder Brequinar.

Bei den Arzneimitteln (= pharmazeutischen Präparaten) handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, dem Alter und dem individuellen Zustand, individuellen pharmakokinetischen Gegebenheiten, der zu behandelnden Erkrankung sowie der Applikationsweise ab.

Die Dosis der verwendeten Wirkstoffe, welche die Konzentration an Pyrimidinbasen-Bausteinen für die Nukleinsäurebiosynthese im Körper erhöhen, liegt bei Warmblütern, insbesondere beim Menschen, insbesondere im Bereich zwischen 1 mg und 15 g pro m² Körperoberfläche und Tag; für Uridin-Phosphorylaseinhibitoren liegt die Konzentration vorzugsweise im Bereich von 10 mg bis 1 g pro Tag, verteilt auf 1 bis 3 Dosen; für Hemmstoffe der Uridinsekretion liegt die Dosis vorzugsweise im Bereich zwischen 1 bis 5 mg/kg Körpergewicht ein-bis dreimal täglich; für Verbindungen, die mit Uridin bei renalen Transportmechanismen kompetitieren, liegen die verabreichten Dosen vorzugsweise im Bereich von 5 bis 50 mg/kg Körpergewicht, verabreicht ein- bis dreimal täglich.

Die Pyrimidinnukleoside und/oder Prodrugs davon werden vorzugsweise in solchen Dosen verabreicht, dass sich Blutplasmakonzentrationen zwischen 10 und 500 µM, insbesondere zwischen 20 und 300 µm, vor allem zwischen 25 und 250 µM, z.B. von 50-200 µM einstellen. Die Bestimmung der Blutplasmakonzentrationen erfolgt hierbei nach an sich bekannten Methoden, vorzugsweise unter Verwendung von HPLC, zu bestimmten Zeitpunkten nach der Administration der Pyrimidinnukleoside oder der Prodrugs davon, z.B. 1, 2, 3, 4 und 5 Stunden nach der Zuführung, beispielsweise wie in US 5,583,117 beschrieben.

Vorzugsweise werden Pyrimidinnukleoside und/oder Prodrugs davon, insbesondere Uridin, Cytidin, vor allem Mono-, Di- oder Tri-Acyluridin oder Mono-, Di-, Tri- oder Tetra-Acylcytidin, in Mengen von täglich 10 bis 500 mg/kg Körpergewicht verabreicht, insbesondere zwischen täglich 50 und 300 mg/kg, beispielsweise aufgeteilt in zwei oder drei separate Dosen, die in Abständen zwischen 6 und 12 Stunden eingenommen werden. Die Acylderivate werden bei oraler Verabreichung leichter resorbiert und sind deshalb insbesondere bei oraler Verabreichung bevorzugt.

Die Arzneimittel zur Verwendung gemäß der Erfindung umfassen insbesondere einen oder mehrere der Mitochondrienreaktivatoren, insbesondere der als bevorzugt genannten, zusammen mit pharmazeutisch üblichen Trägermaterialien.

Die Wirkstoffe können z. B. zur Herstellung von pharmazeutischen Präparaten verwendet werden, welche eine wirksame Menge des Wirkstoffes zusammen oder im Gemisch mit einer signifikanten Menge von anorganischen oder organischen, festen oder flüssigen, pharmazeutisch verwendbaren Trägerstoffen enthalten.

Auch wird eine pharmazeutische Zusammensetzung (Präparat) beschrieben, die geeignet ist zur Verabreichung an einen Warmblüter, insbesondere Menschen, zur Behandlung oder Verhütung der vor- und nachstehen genannten Nebenwirkung, insbesondere bei HAART vor allem bei Verabreichung von NRTIs, oder bei der Behandlung mit anderen antiviralen Nukleosidanaloga, umfassend eine zur Verminderung oder Beseitigung der Nebenwirkungen, wirksame Menge eines Mitochondrienreaktivators, zusammen mit wenigstens einem pharmazeutisch annehmbaren Trägermaterial.

Die pharmazeutischen Präparate enthalten von etwa 1 % bis etwa 96 %, vorzugsweise von etwa 20 % bis etwa 90 % eines oder mehrerer Wirkstoffe, welche die Konzentration an Pyrimidinbasen-Bausteinen für die Nukleinsäurebiosynthese im Körper erhöhen (nachstehend als Wirkstoff bezeichnet). Die pharmazeutische Präparate können z. B. in Dosiseinheitsform, wie gebrauchsfertigen Infusionslösungen, Ampullen, Vials, Suppositorien, Dragées, Tabletten oder Kapseln, vorliegen.

Die pharmazeutischen Präparate werden in an sich bekannter Weise, z. B. mittels konventioneller Lösungs-, Lyophilisierungs-, Misch-, Granulier- oder Dragierverfahren, hergestellt.

Vorzugsweise verwendet man für parenterale Anwendung oder Kapseln Lösungen des Wirkstoffes, daneben auch Suspensionen, und zwar insbesondere isotonische wässrige Lösungen oder Suspensionen, wobei diese z.B. bei lyophilisierten Präparaten, welche den Wirkstoff allein oder zusammen mit einem Trägermaterial, z. B. Mannit, enthalten, vor Gebrauch hergestellt werden können. Die pharmazeutischen Präparate können sterilisiert sein und/ oder Hilfsstoffe, z. B. Konservier-, Stabilisier-, Netz- und/oder Emulgiermittel, Löslichkeitsvermittler, Salze zur Regulierung des osmotischen Druckes und/oder Puffer, oder Säuren, z.B. Zitronensäure, enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Hydroxypropylmethylcellulose, Kieselgel, Dextran oder Polyvinylpyrrolidon, enthalten.

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z.B. Laurinsäure, Palmitinsäure, Stearinsäure oder Arachidinsäure, oder entsprechende ungesättigte Säuren, wie z. B. Ölsäure oder Linolsäure, enthalten, gegebenenfalls unter Zusatz von Antioxidantien, wie z.B. Vitamin E, β-Carotin oder 3,5-Di-tert-butyl-4-hydroxytoluol. Die Alkoholkomponente dieser Fettsäureester hat maximal 6 Kohlenstoffatome und ist ein ein- oder mehrwertiger, z.B. ein-, zwei- oder dreiwertiger Alkohol, z. B. Ethanol, Isopropylalkohol,, vor allem aber Glycol und Glycerin. Als Fettsäureester sind daher beispielsweise zu nennen: Ethyloleat, Isopropylmyristat, Isopropylpalmitat, Polyoxyethylenglycerintrioleate, Triglyceride gesättigter Fettsäuren der Kettenlänge C₈ bis C₁₂, besonders aber vegetabile Öle wie Sesamöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gewünschtenfalls granuliert und das Gemisch, gewünschtenfalls nach Zugabe von geeigneten Hilfsstoffen, zu Tabletten, Dragée-Kernen oder Kapseln verarbeitet. Dabei kann man sie auch in Kunststoffträger einbauen, welche die Wirkstoffe dosiert abgeben oder diffundieren lassen.

Geeignete Trägerstoffe sind insbesondere Füllstoffe, wie Zucker, z. B. Lactose, Saccharose, Mannit oder Sorbit, Cellulosepräparate, oder Calciumphosphate, ferner Bindemittel, wie Stärkekleister unter Verwendung z. B. von Mais- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium-oder Calciumstearat, und/oder Polyethylenglycol. Dragée-Kerne werden mit geeigneten, gegebenenfalls magensaftresistenten Überzügen versehen.

Kapseln sind Steckkapseln aus Gelatine sowie weiche, geschlossene Kapseln aus Gelatine und einem Weichmacher, wie Glycerin oder Sorbit. Die Steckkapseln können den Wirkstoff in Form eines Granulats, z. B. mit Füllstoffen, wie Lactose, Bindemitteln, wie Stärken, und/oder Gleitmitteln, wie Talk oder Magnesiumstearat, und gegebenenfalls mit Stabilisatoren, enthalten. In Kapseln ist der Wirkstoff vorzugsweise in geeigneten öligen Hilfsstoffen, wie fetten Ölen, Paraffinöl oder flüssigen Polyethylenglykolen, gelöst oder suspendiert, wobei ebenfalls Stabilisatoren und/oder antibakterielle Mittel zugefügt sein können. Stabilisatoren, wie Emulgatoren, Netzmittel oder Tenside, Bindemittel, wie Stärkekleister Traganth, Methylcellulose, Hydroxypropylmethylcellulose oder Hydroxypropylcellulose, Natrium-carboxymethylcellulose, Cyclodextrin(e) und/ oder Polyvinylpyrrolidon, und/oder antibakterielle Mittel können zugefügt sein. Als Emulgatoren kommen insbesondere Ölsäure, nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern, wie Polyoxyethylen-sorbitanmonolaurat, -oleat, - stearat, -palmitat, -tristearat oder -trioleat, Polyethylenglycol-Fettsäureester wie Polyoxyethylstearat, Polyoxyethylenglycol-(300 oder 400)-stearat, Polyethylenglycol-2000-stearat, oder Ethylenoxid-Propylenoxid-Blockpolymere, in Frage.

Den Tabletten oder Dragee-Überzügen oder Kapselhüllen können Farbstoffe oder Pigmente, z.B. zur Identifizierung oder Kennzeichnung verschiedener Wirkstoffdosen, beigefügt werden.

Die Verabreichung erfolgt parenteral, wie intravenös (beispielsweise durch i.m., s.c. oder insbesondere i.v. Injektion, durch Infusion oder, insbesondere bei Auftreten von toxischen Symptomen wie Phlebitis einer peripheren Vene, per zentralem Venenkatheter), insbesondere bei akuter Laktazidose oder Leberversagen, sonst enteral, insbesondere oral.

Die Erfindung betrifft auch die Verwendung der Kombinationen von (A) Wirkstoffen, welche die Konzentration an Pyrimidinbasen-Bausteinen für die Nukleinsäurebiosynthese im Körper erhöhen, mit (B) einem oder mehreren anderen Wirkstoffen, insbesondere mit antiviralen Wirkstoffen, wie solchen zur HAART, vorzugsweise NRTIs, oder mit zur Behandlung von Infektionen mit anderen Viren, insbesondere den oben genannten, verwendeten anderen antiviraler Nukleosidanaloga. Insbesondere betrifft die Erfindung die Verwendung der Kombinationen oder Produkte, welche die unter (A) genannten Wirkstoffe und einen oder mehrere der unter (B) genannten Wirkstoffe entweder in fester Kombination oder zur zeitlich gestaffelten oder gleichzeitigen Anwendung in einem gemeinsamen Kit enthalten.

Auch für die Behandlung der Nebenwirkungen in Behandlungsintervallen der antiviralen Therapie (Unterbrechung der HAART, insbesondere Unterbrechung der Therapie mit NRTIs , oder mit anderen Nukleosidanalogen) eignen sich solche Produkte, bei denen die Therapeutika in einem gemeinsamen Kit mit den Mitochondrienreaktivator enthaltenden Präparaten vorliegen.

Neben den genannten Hemmern der Biosynthese von Nukleinsäuren oder deren Vorstufen können weitere Therapeutika, insbesondere zur Behandlung von AIDS, gleichzeitig oder in zeitlich gestaffelter Weise verwendet werden, wie Entry-Inhibitoren, CD4-Bindungsinhibitoren, Chemokinrezeptor-Antagonisten, HIV-Proteasehemmer, CXCR-4-Antagonisten, Fusions-Inhibitoren, Nicht-Nukleosidanaloge RT-Hemmer (z.B. MKC-442 (Emivirin), SJ-3366 (Samjin Pharmaceuticals), TMC120 (Tibotec), UC-781 (Uniroyal), PNU-242721 (Pharmacia & Upjohn, Calanolide-A (SarawakMed), DPC-963,083 (DuPont), UIC-94-003, AG-1549 (Capravirin; Agouron), GW420867X (Glaxo-Wellcome), DPC-961 (DuPont)), Pyrophosphat-Analoge,Integrase-Hemmer, Zinkfinger-Inhibitoren, Interferone und tat/Rev-Inhibitoren; sowie ferner weitere Therapeutika, wie Antibiotika (beispielsweise zur Therapie von Sekundärinfektionen bei AIDS) oder andere Chemotherapeutika. Entsprechende pharmazeutische Präparate können ebenfalls Kits sein, in denen mehrere Wirkstoffe zur gleichzeitigen oder zeitlich gestaffelten Administration nebeneinander als pharmazeutische Formulierungen vorliegen, oder als fixe Kombinationen vorliegen.

### Bevorzugte Ausführungsformen der Erfindung

Bevorzugte Ausführungsformen der Erfindung lassen sich durch Einsetzen spezifischerer Definitionen für allgemeine Ausdrücke und Symbole in den obigen Ausführungsformen erhalten. Weitere bevorzugte Ausführungsformen finden sich in den Unteransprüchen.

### Beispiele:

Die nachfolgenden Beispiele dienen der Erläuterung der Erfindung,

### Beispiel 1: Kompensation der ddC-bedingten mtDNS-Verarmung in Leberzelllinien durch Uridin

*Zellkultur*. Die HepG2-Zelllinie, eine humane Hepatom-Zelllinie, wird von der American Type Culture Collection bezogen (ATCC No. HB-8065) Sie wird bei 37 °C in einer 5%-igen CO₂-Atmosphäre kultiviert. Bei dem Kulturmedium handelt es sich um Dulbecco's Modified Eagle Medium (DMEM - beinhaltet 4,5 g/l Glukose, 110 mg/l Pyruvat, 4 mg/l Nicotinamid, 4 mg/l Pyridoxal-hydrochlorid, 4 mg/l Thiamin und 0,4 mg/l Riboflavin), ergänzt mit 10 % foetalem Rinderserum (50 ml FCS, Mycoplex PAA Laboratories, Linz). 2,7 x 10⁶ Zellen werden am Tag 1 in 75 ml-Kulturflaschen (Falcon, Becton Dickinson, USA) eingebracht. An den Tagen 5, 10, 15, 20 und 25 werden die Zellen trypsinisiert und gezählt, 2,7 x 10⁶ Zellen werden jeweils erneut ausplatiert. Das Medium wird jeweils an den Tagen des Trypsinverdaus und am dritten Tag nach dem erneuten Ausplattieren gewechselt.

*Materialien*: Riboflavin (Vitamin B₂), Thiamin (Vitamin B₁), Ascorbinsäure (Vitamin C), Uridin und ddC werden von Sigma (USA) gekauft. Die Konzentrationen für Uridin werden in den nachfolgenden Tabellen erwähnt, ddC wird in einer Konzentration von 177 nM eingesetzt, was den Gleichgewichts-Plasmaspiegeln von Patienten unter HAART entspricht. Ein Vitamin-Cocktail, der aus 7,125 mg/L Riboflavin, 7,125 mg/l Thiamin und 142,5 mg/l Vitamin C besteht, wird verwendet. Diese pharmakologischen Konzentrationen überschreiten den Tagesbedarf um etwa das hundertfache und sind ausgerichtet an den oberen sicheren Grenzwerten nach den Empfehlungen der Europäischen Union für diese Mikronährstoffe..

*Ermittlung derintrazellulären Lipide*: Intrazelluläre Lipidtröpfchen werden mittels Oil-Red-O (Sigma, USA) angefärbt. Die Zellen werden auf Deckgläschen platiert, mit Oil-Red-O während 30 min inkubiert und mit Meyer's Hämatoxylin (8 min) gegengefärbt, dann mit Wasser gewaschen und in Glycerin-Gelatine eingebettet.

*Milchsäure*: 1,5 ml Kulturüberstand wird jeweils unmittelbar vor der Trypsinisierung entnommen und darin das L-Laktat enzymatisch mit dem Laktat-Testkit von Roche Diagnostics (Best.-Nr. 1822837) in einem automatisierten Analysegerät (Roche/Hitachi 917) nach Herstellerangaben bestimmt.

*Quantifizierung mitochondrialer DNS (mtDNS) durch Southern Blot*: Der Gehalt an mtDNS wird mittels einer bekannten Southern-Blot-Technik ermittelt (vgl. C.T. Moraes et al, "Quantitative Defects of Mitochondrial DNA", in: S. DiMauro and D.C. Wallace, Mitochondrial DNA in Human Pathology", Raven Press, New York 1993, Chapter 8, pages 97-108). Genomische DNS wird aus Fettgewebe extrahiert und mit der Restriktionsendonuklease *Pvull* geschnitten. Fünf µg des geschnittenen Materials werden auf einem 0,8 %-igen Agarosegel elektrophoretisch getrennt und auf eine Nylonmembran übertragen. Die mtDNS wird mit einem 12,9 kBp mtDNS Fragment (welches die Nukleotidpositionen 3470 und 16379 humaner mtDNS umfasst (vgl. S. Anderson et al., Nature 9, 457-65 (1981) und welches mittels stochastisch bindenden Primem mit Digoxigenin-markiert ist), sondiert. Um einen internen Standard zu haben, wird gleichzeitig mit einer zweiten Digoxigenin-konjugierten Sonde markierte Kern-DNS (nDNS), die an das in hoher Kopienzahl vorliegende 18S-ribosomale DNS-Gen hybridisiert, als Sonde eingesetzt. Die Signale für mtDNS und nDNS werden mit einem monoklonalen Antikörper gegen Digoxigenin, der an alkalische Phosphatase konjugiert ist, nach den Herstellerangaben visualisiert (Boehringer Mannheim, Deutschland). Die Signalintensitäten werden per Computer mittels Standard-Software quantifiziert. Der mtDNS-Gehalt wird als mtDNS/nDNS (M/N)-Verhältnis ausgedrückt.

*Ergebnisse:* Außer während einer kurzen Verzögerungsphase nach der Trypsinisierung zeigen die HepG2-Zellen während der gesamten, 25-tägigen Inkubationszeit logarithmisches Wachstum.

Uridin in einer Konzentration von 200 µM normalisiert das Überleben von HepG2-Zellen in Gegenwart von ddC, wie aus Tab. 1 ersichtlich ist:

**Tabelle 1: Zellzahl (in 100.000) am Tag der Trypsinierung (Tag 0 = 100%)**

| | **Tag 0** | **Tag 5** | **Tag 10** | **Tag 15** | **Tag 20** | **Tag 25** |
|---|---|---|---|---|---|---|
| Hep G2-Zellen ohne NRTI, ohne Uridin | - | 129,2 | 121,5 | 118,5 | 121,5 | 127,4 |
| | | (SD = | (SD = | (SD = | (SD = | (SD = |
| | | 15,8) | 12,3) | 13,7) | 7,9) | 13,8) |
| ddC 177nM | - | 110,0 | 98,5 | 92,1103 | 66,2 | 38,4 |
| | | | | ,0 | | |
| ddC 177nM + Uridin 200 µM | - | 122,4 | 132,0 | 114,4 | 120,2 | 130,6 |
| ddC 177nM + Vitamine | - | 124,0 | 106,4 | 58,8 | 43,4 | - |

| | | | | | | |
|---|---|---|---|---|---|---|
| **SD = Standardabweichung (n = 7)** | | | | | | |

Uridin schwächt die Verminderung der mtDNA ab, siehe Tab. 2

**Tabelle 2: Gehalt mitochondrialer DNS, ausgedrückt als M/N-Verhältnis in % der Kontrolle (HepG2-Zellen ohne ddC und ohne Uridin an Tag 0):**

| | **Tag 0** | **Tag 5** | **Tag 10** | **Tag 15** | **Tag 20** | **Tag 25** |
|---|---|---|---|---|---|---|
| ddC 177nM | 100,0 | 25,6 | 25,1 | 11,5 | 8,3 | 8,4 |
| ddC 177nM + Uridin 200 µM | 100,0 | 74,7 | 60,4 | 64,2 | 53,1 | 67 |
| ddC 177nM + Vitamine | 100,0 | 16,0 | 8,9 | 9,7 | 9,3 | - |

| | | | | | | |
|---|---|---|---|---|---|---|
| **n.d. = nicht gemessen.** | | | | | | |

Die Produktion von Milchsäure, die als Ergebnis vermehrter kompensatorischer Glykolyse und eines zugunsten von NADH verschobenen NADH/NAD-Gleichgeichtes wegen der Hemmung der mitochondrialen oxidativen Phosphorylierung durch den Hemmer der gamma-Polymerase auftritt, wird durch Uridin abgeschwächt, siehe Tab. 3.

**Tabelle 3: Laktatwerte im Medium in Prozent der Kontrolle (ohne ddC und ohne Uridin) - Tag 0 = 100%**

| | **Tag 0** | **Tag 5** | **Tag 10** | **Tag 15** | **Tag 20** | **Tag 25** |
|---|---|---|---|---|---|---|
| ddC 177nM | - | 109,2 | 129,5 | 220,1 | 374,9 | 392,5 |
| ddC 177nM + Uridin 200 µM | - | 101,5 | 99,1 | 100,7 | 106,6 | |
| ddC 177nM + Vitamine | - | 196,1 | 143,0 | 249,0 | 302,1 | - |

Bei Messung der intrazellulären Lipide zeigt sich am Tag 20 eine deutliche Verringerung der steatotischen Veränderungen in Gegenwart von Uridin. Während in Gegenwart von 177 µM ddC und 200 µM Uridin das mikroskopische Bild dem der Kontrolle ohne ddC und ohne Uridin gleicht (viele Zellen, wenig Rotfärbung durch Oil-Red-O), werden in Gegenwart von 177 µM ddC (ohne Uridin) deutlich weniger Zellen gefunden, die jedoch relativ groß und rot gefärbt (= fetthaltig) sind. In Gegenwart von 177 µM ddC und des Vitamin-Cocktails ähnelt das erhaltene Bild demjenigen bei Anwesenheit von 177 µM ddC ohne Uridin. Somit ist lediglich die Anwesenheit von Uridin geeignet, die Nebenwirkungen von ddC zu kompensieren. Die Supplementierung mit Vitaminen dagegen zeigt keinen erkennbaren günstigen Effekt (vgl. auch Tab. 1 bis 3).

### Beispiel 2: Kompensation der NRTI-bedingten mtDNS-Verarmung in Leberzelllinien durch Uridin

Analog Beispiel 1 werden Versuche mit ddl bzw. mit d4T anstelle von ddC durchgeführt.

### Beispiel 3: Formulierungsbeispiel für Infusionslösung mit Uridin:

In einer Konzentration von 10 mg/ml wird Uridin in 100 ml 0,9%-iger Kochsalzlösung gemäß US Pharmacopoeia unter sterilen Bedingungen verdünnt und die resultierende Infusionslösung zur Infusion verwendet.

### Beispiel 4: Tabletten mit Triacetyluridin

Die folgenden Ingredientien werden zur Herstellung von 5000 Tabletten, die pro Stück 200 mg Triacetyluridin (Herstellung siehe US 5,538,117) enthaltend, verwendet:

| | |
|---|---|
| Triacetyluridin | 1000 g |
| Maisstärke | 680 g |
| Kolloidales Kieselgel | 200 g |
| Magnesiumstearat | 20 g |
| Stearinsäure | 50 g |
| Natriumcarboxymethylstärke | 250 g |
| Wasser | quantum satis |

Eine Mischung von Triacetyluridin, 50 g Maisstärke und das kolloidale Kieselgel wird zusammen mit Stärkepaste, die aus 250 g Maisstärke und 2,2 kg entmineralisiertem Wasser, verarbeitet, wobei eine feuchte Masse entsteht. Diese wird durch ein Sieb mit 3 mm Maschengröße gepresst und in einem Wirbelschichttrockner bei 45 °C während 30 min getrocknet. Das getrocknete Granulat wird durch ein Sieb mit 1 mm Maschengröße gepresst, mit einer zuvor durch ein 1 mm-Sieb passierten Mischung von 330 g Kornstärke, dem Magnesiumstearat, der Stearinsäure und der Natrium-carboxymethylcellulose gemischt und in leicht gewölbte Tabletten verpresst.

## Patentansprüche

1. Verwendung eines Pyrimidinnukleosids oder eines Prodrugs eines Pyrimidinnukleosids zur Herstellung eines Medikamentes zur Behandlung oder Prävention der Lipodystrophie, wobei das Prodrug einer Pyrimidin Orotsäure, Uracil, Cytosin oder Thymin oder das Prodrug einer der nachstehend gezeigten Formeln I oder II ist 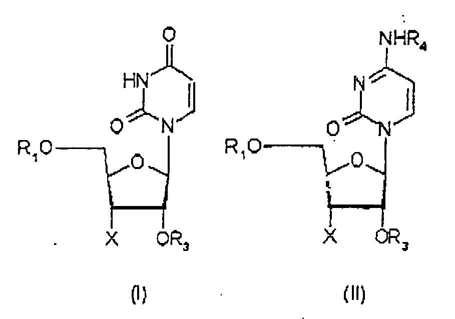 , worin X Wasserstoff oder OR₂ ist und R₁, R₂, R₃ und, falls vorhanden R₄, unabhängig Wasserstoff oder Acyl ist, wobei mindestens einer der Reste der R₁, R₂, R₃ und R₄ Acyl ist.

2. Verwendung einer Kombination eines Pyrimidinnukleosids oder eines Prodrugs eines Pyrimidinnukleosids mit Hemmern der Biosynteste von Nucleinsäuren zur Herstellung eines Kombinationspräparats zur Behandlung oder Prävention der Lipodystrophie, wobei das Prodrug einer Pyrimidin Orotsäure, Uracil, Cytosin oder Thymin oder das Prodrug einer der nachstehend bezeigten Formeln I oder II ist, worin X Wasserstoff oder OR₂ ist und R₁, R₂, R₃ und, falls vorhanden R₄, unabhängig Wasserstoff oder Acyl ist, wobei mindestens einer der Reste R₁, R₂, R₃ und R₄ Acyl ist.

3. Verwendung gemäß einem der Ansprüche 1 oder 2, wobei Lipodystrophie NRTI-assoziierte Lipodystrophie, NRTI-assoziierte Dyslipidemia und durch Fettstoffwechsel verursachte NRTI-assoziierte Insulinresistenz umfasst.

4. Verwendung gemäß einem der Ansprüche 1 oder 2, wobei das Pyrimidinnukleosid Uridin ist.

5. Verwendung gemäß einem der Ansprüche 1 bis 4, gleichzeitig mit der Einnahme eines Hemmers der Biosynthese von Nukleinsäuren.

6. Verwendung gemäß Anspruch 5, wobei die Hemmer der Biosynthese von Nukleinsäuren Nukleosidanaloge Reverse-Transkiptase-Hemmer (NRTI's) sind.

## Claims

1. Use of a pyrimidine nucleoside or a prodrug of a pyrimidine nucleoside for production of a drug for treatment or prevention of lipodystrophy, wherein the prodrug is a pyrimidine orotic acid, uracil, cytosine or thymine, or the prodrug has one of the following formulas 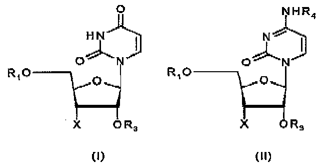 where X is hydrogen or OR₂, and R₁, R₂, R₃ and, if present, R₄, are independent hydrogen or acyl, wherein at least one of the residues of R₁, R₂, R₃ and R₄ is acyl.

2. Use of a combination of a pyrimidine nucleoside or a prodrug of a pyrimidine nucleoside with inhibitors of the biosynthesis of nucleic acids for production of a combination drug for treatment or prevention of lipodystrophy, wherein the prodrug is a pyrimidine orotic acid, uracil, cytosine or thymine, or the prodrug has one of the above formulas, wherein X is hydrogen or OR₂, and R₁, R₂, R₃ and, if present, R₄, are independent hydrogen or acyl, wherein at least one of the residues of R₁, R₂, R₃ and R₄ is acyl.

3. Use according to claim 1 or 2, wherein lipodystrophy comprises NRTI-associated lipodystrophy, NRTI-associated dyslipidemia and NRTI-associated insulin resistance caused by fat metabolism.

4. Use according to claim 1 or 2, wherein the pyrimidine nucleoside is uridine.

5. Use according to one of the claims 1-4, simultaneously with the intake of an inhibitor of the biosynthesis of nucleic acids.

6. Use according to claim 5, wherein the inhibitors of the biosynthesis of nucleic acids are nucleoside-analogous reverse transcriptase inhibitors (NRTI's).

## Revendications

1. Utilisation d'un nucléoside pyrimidique ou d'un pro-médicament d'un nucléoside pyrimidique pour la production d'un médicament pour le traitement ou la prévention de la lipodystrophie, où le pro-médicament d'une pyrimidine est l'acide orotique, l'uracile, la cytosine ou la thymine ou est le pro-médicament de l'une des formules I ou II montrées dans la suite 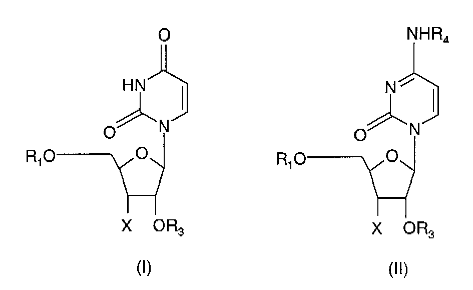 où X est l'hydrogène ou OR₂ et R₁, R₂, R₃ et, s'il est présent, R₄ sont indépendamment l'hydrogène ou acyle, où au moins l'un des groupements de R₁, R₂, R₃ et R₄ est acyle.

2. Utilisation d'une combinaison d'un nucléoside pyrimidique ou d'un pro-médicament d'un nucléoside pyrimidique avec des inhibiteurs de la biosynthèse des acides nucléiques pour la production d'une préparation combinée pour le traitement ou la prévention de la lipodystrophie, où le pro-médicament d'une pyrimidine est l'acide orotique, l'uracile, la cytosine ou la thymine ou est le pro-médicament de l'une des formules I ou II montrées dans la suite, où X est l'hydrogène ou OR₂ et R₁, R₂, R₃ et, s'il est présent, R₄ sont indépendamment l'hydrogène ou acyle, ou au moins l'un des groupements R₁, R₂, R₃ et R₄ est acyle.

3. Utilisation selon l'une des revendications 1 ou 2 où la lipodystrophie comprend la lipodystrophie associée à des NRTI, la dyslipidémie associée à des NRTI et la résistance à l'insuline associée à des NRTI provoquée par le métabolisme des graisses.

4. Utilisation selon l'une des revendications 1 ou 2 où le nucléoside pyrimidique est l'uridine.

5. Utilisation selon l'une des revendications 1 à 4 en même temps que la prise d'un inhibiteur de la biosynthèse des acides nucléiques.

6. Utilisation selon la revendication 5 où les inhibiteurs de la biosynthèse des acides nucléiques sont des inhibiteurs de transcriptase inverse analogues de nucléosides (NRTI).
